# EUROPEAN PATENT SPECIFICATION

(11) **EP 4 012 036 B1**
(45) Date of publication and mention of the grant of the patent: **20.11.2024**
(21) Application number: 20848947.6
(22) Date of filing: 24.04.2020
(51) Int. Cl.: C12N 15/867, C12N 5/10

(54) **LIVER PRECURSOR LIKE CELL LINE, CONSTRUCTION METHOD, AND APPLICATION TO FIELD OF BIOARTIFICIAL LIVERS**
LEBERVORLÄUFER-ÄHNLICHE ZELLLINIE, HERSTELLUNGSVERFAHREN UND ANWENDUNG AUF DEM GEBIET DER BIOARTIFIZIELLEN LEBER
LIGNÉE CELLULAIRE DU TYPE PRÉCURSEUR HÉPATIQUE, PROCÉDÉ DE CONSTRUCTION ET APPLICATION AU DOMAINE DES FOIES BIO-ARTIFICIELS

(30) Priority: 05.08.2019 CN 201910718292
(43) Date of publication of application: 15.06.2022
(73) Proprietor: Shanghai Celliver Biotechnology Co., Ltd., Shanghai 201210 (CN); Shanghai Cryowise Medical Technology Co., Ltd., Shanghai 201210 (CN)
(72) Inventor: YAN, Hexin, Shanghai 201203 (CN)
(74) Representative: Bayramoglu et al.
(86) International application number: PCT/CN2020/086660
(87) International publication number: WO 2021/022839

(56) References cited:
- WO-A1-2005/085422
- CN-A- 103 981 147
- CN-A- 109 207 429
- CN-A- 109 337 858
- CN-A- 110 438 157
- US-A1- 2017 246 215
- PENGYU HUANG ET AL: "Direct Reprogramming of Human Fibroblasts to Functional and Expandable Hepatocytes", CELL STEM CELL, vol. 14, no. 3, 1 March 2014 (2014-03-01), pages 370 - 384, XP055194338, ISSN: 1934-5909, DOI: 10.1016/j.stem.2014.01.003
- FU GONG-BO ET AL: "Expansion and differentiation of human hepatocyte-derived liver progenitor-like cells and their use for the study of hepatotropic pathogens", CELL RESEARCH, SPRINGER SINGAPORE, SINGAPORE, vol. 29, no. 1, 25 October 2018 (2018-10-25), pages 8 - 22, XP036667414, ISSN: 1001-0602, [retrieved on 20181025], DOI: 10.1038/S41422-018-0103-X
- DAI KEQIANG ET AL: "Induction of Functional Hepatocyte-Like Cells by Overexpression of FOXA3 and HNF4[alpha] in Rat Bone Marrow Mesenchymal Stem Cells", CELLS TISSUES ORGANS, vol. 200, no. 2, 1 January 2014 (2014-01-01), CH, pages 132 - 140, XP055970403, ISSN: 1422-6405, Retrieved from the Internet <URL:https://www.karger.com/Article/Pdf/380762> DOI: 10.1159/000380762
- WEGE HENNING ET AL: "Telomerase reconstitution immortalizes human fetal hepatocytes without disrupting their differentiation potential", GASTROENTEROLOGY, ELSEVIER INC, US, vol. 124, no. 2, 1 February 2003 (2003-02-01), pages 432 - 444, XP002395850, ISSN: 0016-5085, DOI: 10.1053/GAST.2003.50064
- HANG HUALIAN , ZHANG LEI , SHI XIAOLEI: "Establishment and biological study of immortalized human hepatocyte cell line", JIANGSU MEDICAL JOURNAL, vol. 37, no. 22, 30 November 2011 (2011-11-30), pages 2624, XP055778351, ISSN: 0253-3685, DOI: 10.19460/j.cnki.0253-3685.2011.22.006
- MAI G; HUY N T; MOREL P; MEI J; BOSCO D; BERNEY T; MAJNO P; MENTHA G; TRONO D; BUHLER L H: "Treatment of Fulminant Liver Failure by Transplantation of Microencapsulated Primary or Immortalized Xenogeneic Hepatocytes", TRANSPLANTATION PROCEEDINGS, vol. 37, no. 1, 31 December 2005 (2005-12-31), pages 527 - 529, XP004820005, ISSN: 0041-1345, DOI: 10.1016/j.transproceed.2005.01.017

## Description

### BACKGROUND

### Technical Field

The present invention relates to the field of the biotechnology, particularly relates to a hepatic precursor-like cell line, a production method, and applications in the field of the bioartificial livers.

### Description of Related Art

Liver failure is the end stage manifestation of severe liver disease, and the fatality rate of the patients can be as high as 50% to 90%. Liver transplantation is an effective method for the treatment of liver failure. However, because of the high expense, lack of donors, serious immune rejection, and other problems, liver transplantation is difficult to carry out extensively. Currently, there is certainly progress and technological breakthroughs have been made in the research of hepatocyte transplantation and bioartificial liver technology, which is expected to become an effective treatment for liver failure.

The Chinese invention patent application with the publication number CN105521482A discloses the combined application of the different types of the hepatocyte-specific transcription factors, such as HNF1α, HNF4α or FOXA3 to induce differentiation of the hepatocellular carcinoma cells. The patent reveals the above-mentioned hepatocyte-specific transcription factors in the differentiation therapy of liver cancer have good application prospects.

The Chinese invention patent application with the publication number CN103981147A discloses a method for preparing hepatocyte-like cells. This method induces differentiated somatic cells into functional hepatocytes in vitro by overexpressing several transcription factors in somatic cells such as fibroblasts. However, the seed cells of the method are derived from non-hepatic tissues, so only through the reprogramming does not guarantee that it can completely achieve hepatic differentiation, which increases the culture cost of inducing differentiation.

As a result, it is necessary to develop a new type of hepatic precursor-like cell line and its construction to avoid the above-mentioned problems in the prior art.

### SUMMARY

The purpose of this invention is to provide a hepatic precursor-like cell line that can easily accomplish good liver differentiation, a production method thereof, and an application in the field of bioartificial liver, so as to reduce the cost of culture.

In order to carry out the above purposes, the method for producing the hepatic precursor-like cell line of this invention comprises:
S1: providing human primary hepatocyte cultures, proliferation-and passage-culturing the human primary hepatocyte cultures with a passage ratio of 1:3 to 1:6 and a passage number of 2 to 30 to obtain to-be-infected cultures;
S2: confluence-culturing the to-be-infected cultures at a seeding density of 0.5×10⁴-1×10⁵ cells/cm² to obtain adherent cells, wherein the confluence rate of the adherent cells is not less than 80%;
S3: virus-infecting the adherent cells with a lentivirus suspension and a viral infection enhancement buffer using TEM medium, wherein one expressed gene of the lentivirus is SV40 large T antigen, HPV E6E7 gene or human telomerase reverse transcriptase gene (hTERT gene), and the viral infection enhancement buffer is polybrene buffer, and replacing the TEM medium during the virus infection, wherein the amount of the lentivirus in the suspension is 0.5 to 50 times the amount of adherent cells, and the concentration of the virus infection enhancement buffer is 6-12 µg/ml;
S4: proliferation-culturing and subculturing the infected cells with a selecting agent and an amplifying medium for a proliferation duration of 24-36 hours to obtain immortalized cells with a subculture ratio of 1:3 to 1:6, and a passage number of 10 to 100, wherein the selecting agent is puromycin, hygromycin, or neomycin sulfate and used for selection of the infected cell, and the amplification medium is the TEM medium; and
S5: overexpressing FOXA3 in the immortalized cell line to obtain the hepatic precursor-like cell line.

The present invention also provides a hepatic precursor-like cell line prepared by the production method. The hepatic precursor-like cell line is a viable sample deposited in the China Center for Type Culture Collection in Wuhan University, No. 299 Bayi Road, Wuchang District, Wuhan Hubei Province, China. The preservation number is CCTCC NO.: C2019120, and the classification is named ALI-CELL-85F.

Specifically, the culture is received by the China Center for Type Culture Collection on June 11, 2019 and is named as immortal human hepatic progenitor cell-like cell line ALI-CELL-85F. The culture is tested and identified as a viable state on June 24, 2019. The immortal human hepatic progenitor cell-like cell line ALI-CELL-81.5 is the immortal hepatic progenitor cell-like cell line having a category name of ALI-CELL-85F according to this invention.

The China Center for Type Culture Collection shall, upon request, store the culture for 30 years starting from June 11, 2019, and shall continue to store it for another 5 years after receiving the request to provide culture samples before the expiration date.

The beneficial effect of the production method of this invention is that since the primary hepatocyte cultures are derived from liver tissues, the primary hepatocytes in the primary hepatocyte cultures are used as seed cells to construct the hepatic precursor-like cell line, which combines with controlling the technical conditions of the passage, the subculture, the virus infection, the proliferation culture, and the confluence culture, as well as overexpresses the hepatocyte-specific transcription factor in the infected cells to obtain the hepatic precursor-like cells line which is easily achieve good liver differentiation.

Preferably, in the step S5, the hepatocyte-specific transcription factor is Forkhead Box A3, FOXA3.

Preferably, in the step S1, a collagenase perfusion method is used to separate the primary hepatocyte cultures from the normal liver tissues.

Preferably, in the step S1, the primary hepatocyte cultures are proliferation-cultured at a seeding density of 0.5×10⁴-2×10⁴ cells/cm² for 6 to 12 days. The beneficial effect thereof is that controlling an appropriate seeding density is helpful to promote the growth in a shorter time and reproduction of the primary cells and creates good conditions for the subsequent subculture.

Further preferably, after the coating buffer is coated in the culture dish with a coating density of 0.87-1.74 µl/cm², the primary cell cultures are seeded in the culture dish, and then the TEM medium is used for the proliferation culture.

Preferably, in the step S2, the to-be-infected cells are confluence-cultured with the coating buffer coated in the culture dish at a coating density of 0.87-1.74 µl/ cm² for at least 24 hours. The beneficial effect is that controlling the coating density of the coating buffer is helpful to enhance the adherence ability of the to-be-infected cells to be reached a good confluence rate in a short time.

Further preferably, in the step S3, the lentivirus suspension and the viral infection enhancement buffer are added into the culture dish with the adherent cells, and after 6 to 12 hours, the coating buffer is replaced. The beneficial effect is beneficial to promote the effect of the virus infection further.

Further preferably, after the coating solution is replaced, the adherent cells are continuously cultured for 24 to 72 hours to complete the virus infection.

Further preferably, cultures obtained by the virus infection are performed a fluorescent analysis to count the fluorescent rate, when the fluorescent rate is higher than 90%, the step S4 is performed. The beneficial effect is to ensure the cell obtained after virus infection with a high infection efficiency, which makes for creating the good conditions for the subsequent construction of the immortalized cell line.

### BRIEF DESCRIPTION OF THE DRAWINGS

FIG. 1 is a schematic diagram of the morphology of the hepatic precursor-like cell line obtained by the first construction method of this invention.
FIG. 2 is a comparison diagram of the gene expression levels of the different functional genes of the control cell line and the first target cell line of this invention.
FIG. 3 is a comparison diagram of the ammonia clearance capacity of the control cell line and the first target cell line of this invention.
FIG. 4 is a comparison diagram of the α1-antitrypsin level of the control cell line and the first target cell line of this invention.
FIG. 5 is a comparison diagram of the Western blotting of the control cell line and the second target cell line of this invention.
FIG. 6 is a comparison diagram of the urea production level of the control cell line and the third target cell line of this invention.
FIG. 7 is a comparison diagram of the albumin level of the control cell line and the third target cell line of this invention.
FIG. 8 is a comparison diagram of the proliferation performance of the control cell line and the first target cell line of this invention.
FIG. 9 is a comparison diagram of the doubling time of the control cell line and the first target cell line of this invention.
FIG. 10 is a schematic diagram of the structure of the bioartificial liver reactor of this invention.
FIG. 11 is a schematic diagram of the proliferation ability of the hepatocytes seeded on the surface of the blank carriers at different culture times during the gas-liquid interactive culture of the first target cell line of this invention in the bioartificial liver reactor shown in FIG. 10.
FIG. 12 is a schematic diagram of the gene expression level of the different functional genes of the hepatocytes seeded on the surface of the blank carriers this invention after the first target cell line of this invention has been cultured for 12 days in the bioartificial liver reactor shown in FIG. 10.

### DETAILED DESCRIPTION

To make the objectives, technical solutions and advantages of this invention clearer, the technical solutions in the embodiments of this invention will be described clearly and completely with reference to the accompanying drawings of this invention. Obviously, the described embodiments are part of, but not all of, the embodiments of this invention. Unless otherwise defined, the technical or scientific terms used herein shall have the usual meanings understood by those skilled in the art related to this invention. As used herein, "comprising" and other similar terms mean that the elements or objects appearing before the term encompass the elements or objects listed after the term and their equivalents, without excluding other elements or objects.

The main source of the reagents in the embodiments are as follows:
The TEM culture medium, cell suspension of SV40 large T antigen, lentivirus expressing HPV E6E7, and lentivirus expressing the gene of human telomerase reverse transcriptase, hTERT, are from Shanghai Celliver Biotechnology Co., Ltd. The matrigel is produced by Corning Incorporated, and the catalog number is 356234. The polybrene buffer and the neomycin sulfate are produced by Shanghai Yisheng Bio-Technology Co., Ltd, and the catalog numbers are 40804ES79 and 60207ES25 separately. The puromycin is produced by Shanghai Beyotime Biotechnology Co., Ltd., and the catalog number is ST551. FOXA3 is from Shanghai Genechen Co., Ltd. A hygromycin is produced by Thermo Fisher Scientific (Invitrogen), and the catalog number is 10687010.

The sources of the main instruments in embodiments are follows:
Six-well plates are produced by NEST Science Co., Ltd., and the catalog number is 703001. A cell culture incubator is purchased from ESCO Micro Pte. Ltd. Singapore, the catalog number is NO CLL-170B-8. A cell inverted microscope is purchased from Nikon Co., Ltd., and the catalog number is NO Ta2-FL.

In view of the problems in the prior art, embodiments of this invention provides a constructing method of a hepatic precursor-like cell line, including:
S1: providing primary hepatocyte cultures, proliferation-culturing and passage-culturing the primary hepatocyte cultures with a passage ratio of 1:3 to 1:6 and a passage number of 2 to 30 to obtain to-be-infected cultures;
S2, confluence-culturing the to-be-infected cultures at a seeding density of 0.5×10⁴-1×10⁵ cells/cm² to obtain adherent cells, wherein the confluence rate of the adherent cells is not less than 80%;
S3, virus-infecting the adherent cells with a lentivirus suspension and a viral infection enhancement buffer, and replacing a medium during the virus infection, wherein the amount of the lentivirus in the suspension is 0.5 to 50 times the adherent cells, and the concentration of the virus infection enhancement buffer is 6-12 µg/ml;
S4, proliferation-culturing and subculturing the infected cell with a selecting agent and an amplifying medium for 24-96 hours to obtain immortalized cells with a subculture ratio of 1:3 to 1:6 and a passage number of 10 to 100;
S5, overexpressing a hepatocyte-specific transcription factor in the immortalized cell line to obtain the hepatic precursor-like cell line.

Wherein, the coating density is defined as the volume of the coating buffer per unit culture area.

The seeding density is defined as the number of cells per unit area.

The cultures in the same group of the culture dish are separated and reseeded to another culture dish for the subsequent subculture. The number ratio of the pre-separated cultures to the separated cultures is defined as the passage ratio.

In step S5 of some embodiments of this invention, the hepatocyte-specific transcription factor is FOXA3, the sequence thereof is referred to the sequence table.

In other embodiments of this invention, the hepatocyte-specific transcription factor is HNF1α or HNF4α.

In step S1 of some embodiments of this invention, the primary hepatocyte cultures are human primary hepatocyte cultures.

In step S1 of other embodiments of this invention, the primary human hepatocytes are separated from the liver tissues of the normal donor or the normal liver tissues adjacent to hemangioma by a collagenase perfusion method. The collagenase perfusion method and the fluorescence-activated cell sorting are described in the "Expansion and differentiation of human hepatocyte-derived liver precursor-like cells for the study of hepatotropic pathogens" and will not be repeated here.

In step S1 of some embodiments of this invention, after coating the coating buffer with a coating density of 0.87-1.74 µl/cm² in the culture dish, the primary cells are seeded in the culture dish at a density of 0.5×10⁴-2×10⁴ cells/cm² to perform the proliferation culture for 6-12 days.

In step S2 of some embodiments of this invention, the to-be-infected cells are confluence-cultured with the coating buffer coated in the culture dish with a coating density of 0.87-1.74 µl/ cm² for at least 24 hours.

In step S3 of some embodiments of this invention, the lentivirus suspension and the viral infection enhancement buffer are added into the culture dish with the adherent cells, and after 6 to 12 hours, the medium is replaced. After replacing the medium, the adherent cells are continuously cultured for 24 to 72 hours to complete the virus infection.

In step S4 of some embodiments of this invention, the cultures obtained by the virus infection and the selecting agent are mixed and then transferred to an amplifying medium to perform the proliferation culture, and the volume ratio of the selecting agent and the amplifying medium is 1:1000.

In some specific embodiments of this invention, the coating buffer is referred to as Matrigel. The proliferation culture dish and the confluence culture dish are six-well dishes. The medium is the TEM medium. The expressing genes of the lentivirus are SV large T antigen, HPV E6/7 gene, or hTERT. The viral infection enhancement buffer is polybrene buffer. The selecting agent is puromycin, hygromycin, or neomycin sulfate.

The construction method and the advantages of the hepatic precursor-like cell line are described in detail with embodiments 1-3 as follows

### Embodiment 1

The embodiment provides a first construction method.

The six-well culture dishes were used in the embodiment with the same specification, and the Matrigel was added to the six-well culture dish in advance. The coating density of the Matrigel was 0.87 µl/cm².

The incubator was used in the embodiment with a constant temperature of 37 °C, saturated humidity, and a CO₂ concentration of 5%.

In the first construction method, except for the process of culturing the cells in the incubator, other operations were carried out in a laminar airflow bench at room temperature.

Specifically, the first construction method is:
S11: the human primary hepatocytes were seeded in the first culture dish at a density of 1×10⁴ cells/cm². After 2 ml of the TEM medium to each well of the first culture dish was added, the cells were cultured in the incubator for 6 days. As a result, the primary cells in the primary cultures differentiated gradually to obtain the proliferation ability.
   Specifically, the human primary hepatocytes were discarded surgical specimens from Shanghai Eastern Hepatobiliary Surgery Hospital. The patients' consent was obtained and the informed consent forms were signed before the operation.
S12: After completing the proliferation culture, the medium was removed from the first culture dish. The cell culture obtained by the proliferation culture was subcultured with a trypsin digestion solution and a TEM medium with a passage number of 20 and a passage ratio of 1: 3 to obtain the to-be-infected cells.

Specifically, 0.5 ml of trypsin digestion solution and 2 ml of the cultures obtained by the proliferation culture were added to each well of the second culture dish, and the second culture dish was put into the incubator for 3 minutes to perform a digestion-resuspension treatment. After the digestion-resuspension treatment were completed, 2 ml of TEM medium were added to each well of the second culture dish, and the second culture dish was put into the incubator to be cultured. After the bottoms of the second culture dish were observed to be overgrown with the cells, the cells were divided into 3 parts equally. Moreover, 0.5 ml of trypsin digestion solution was added to the other 3 wells to perform the digestion-resuspension treatment for completing a passage. In the process of the passage, 2 ml of TEM medium in each well was replaced with a new TEM medium every 24 hours to improve the passage efficiency.

S21: After the passage was completed, the to-be-infected cells were seeded in the third culture dish at a density of 2×10⁴ cells/cm². The third culture dish was put into an incubator to perform confluence culture. As soon as the confluence rate reached 80 % in the third culture dish was observed, the confluence culture was stopped.

S31: The confluence-cultured cells as mentioned in step S13 were digested and resuspended with trypsin digestion solution to get the digested cultures. The digested cultures were seeded in the fourth culture dish at a density of 2×10⁴ cells/cm², and the fourth culture dish was put into an incubator. After 24 hours later, the supernatant of the fourth culture dish was removed. Then, the TEM medium, SV large T antigen, and polybrene buffer were added into the fourth culture dish, and the cultures were infected for 8 hours in the incubator. After the virus infection was completed, the TEM medium in each well of the fourth culture dish was replaced, and the fourth culture dish was put into the incubator for continuously culturing. After incubating for 72 hours, a small amount of the cell suspension was taken to be analyzed under the fluorescence-activated microscope. If the fluorescence rate was greater than 90 %, the virus infection was completed.

Specifically, 50 µl of the SV large T antigen suspension and 10 µl of the polybrene buffer were added to each well of the fourth culture dish. The ratio of the SV large T antigen's number and the adherent cells' number in the suspension was 20:1. The concentration of the polybrene buffer was 8 µg/ml.

S41: After the virus infection completed, the infected cells were added to the fifth culture dish with the TEM medium and puromycin, and then the fifth culture dish was put into the incubator lasting 24 hours for the proliferation culture.

After the proliferation culture was completed, the TEM medium in the fifth culture dish was replaced with a new TEM medium. Then, the cells in the fifth culture dish were subcultured 20 times with a passage ratio was 1:3 to obtain an immortalized cell line.

Specifically, before the proliferation culture, cells in each well of the fifth culture dish were 0.5 ml, the volume ratio of puromycin and the TEM medium was 1: 1000, and the TEM medium was 2 ml.

One-run specific subculture process was as follows: As soon as the pre-subcultured cells were observed to be overgrown at the bottoms of the culture wells, the pre-subcultured cells were separated into 3 parts. Then the cells were subcultured into the other 3 wells, and 2 ml of a TEM medium was added to each well of the culture dish.

S51: FOXA3 was overexpressed in the immortalized cell line to obtain the viable hepatic precursor-like cell line, which was deposited in the China Center for Type Culture Collection. The preservation number was CCTCC NO. C2019120, and the classification was named ALI-CELL-85F.

Specifically speaking, the implementation of the overexpression was mentioned in "Expansion and differentiation of human hepatocyte-derived liver precursor-like cells for the study of hepatotropic pathogens" published in Cell Research, 2018, 29, 1, and will not be repeated here.

### Embodiment 2

The embodiment provides a second construction method. The difference between the second construction method and the first construction method in Embodimet 1 are:
The coating density of Matrigel was 1.74 µl/cm².

In step S11, the seeding density of the human primary hepatocytes was 2×10⁴ cells/cm².

In step S12, the number of passages was 5, the passage ratio was 1: 4.

In step S21, the seeding density of the to-be-infected cells was 1×10⁵ cells/cm².

In step S31, the ratio of the lentivirus and adherent cells in the suspension was 50: 1. Infection completed, the cells were cultured in the incubator for 48 hours.

In step S41, a TEM medium and hygromycin were added into the fifth culture dish. Moreover, the proliferation culturing was lasted 2 days with a subculture ratio of 1:4 and a subculture number of 40.

### Embodiment 3

The embodiment provides a third construction method. The difference between the third construction method and the first construction method in Example 1 are:
The density of Matrigel was 1 µl/cm².

In step S11, the human primary hepatocytes were proliferation-cultured at a seeding density of 0.5×10⁴ cells/cm² for 3 days.

In step S12, the number of passage was 10, and the passages ratio was 1: 6.

In step S21, the seeding density of the to-be-infected cells was 1.5×10⁴ cells/cm².

In step S31, the lentivirus suspension was expressed the hTERT gene. The density of the digested cells was 1×10⁴ cells/cm², and the virus infection lasted 10 hours. The ratio of the lentivirus and the adherent cells was 40: 1, and the concentration of polybrene buffer was 10 µg/ml.

In step S41, TEM medium and neomycin sulfate were added to the fifth culture dish. Moreover, the proliferation culturing was lasted 4 days with a subculture ratio of 1:6 and a subculture number of 30.

The Embodiments 1-3 of this invention provide the first, the second and the third construction methods, which had good experimental reproducibility.

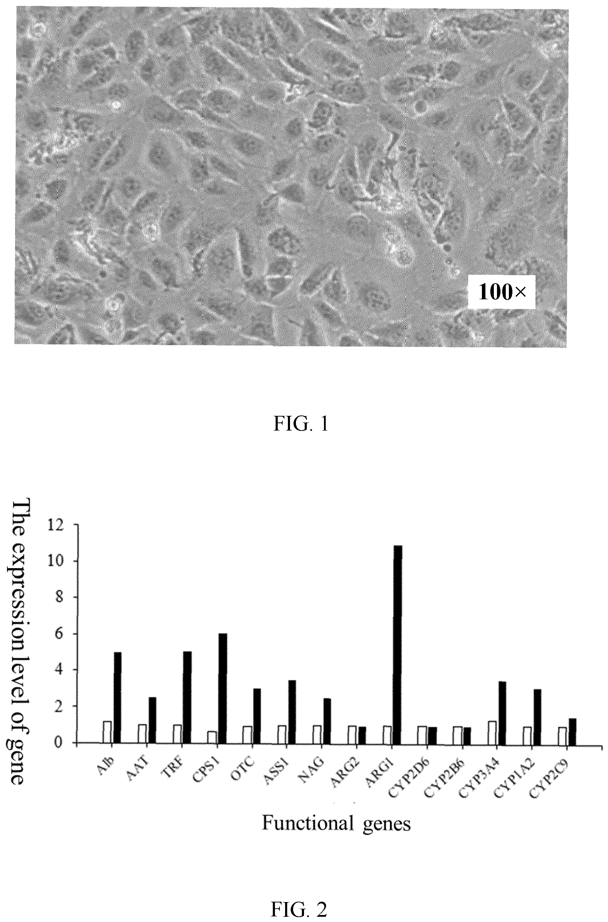

The structure diagram of the morphology of the hepatic precursor-like cell line made by the first construction and observed under a cell inverted microscope at a magnification of 100 times was shown in FIG. 1. The concrete sample preparation and observation methods were ordinary skills in the art and will not be repeated here.

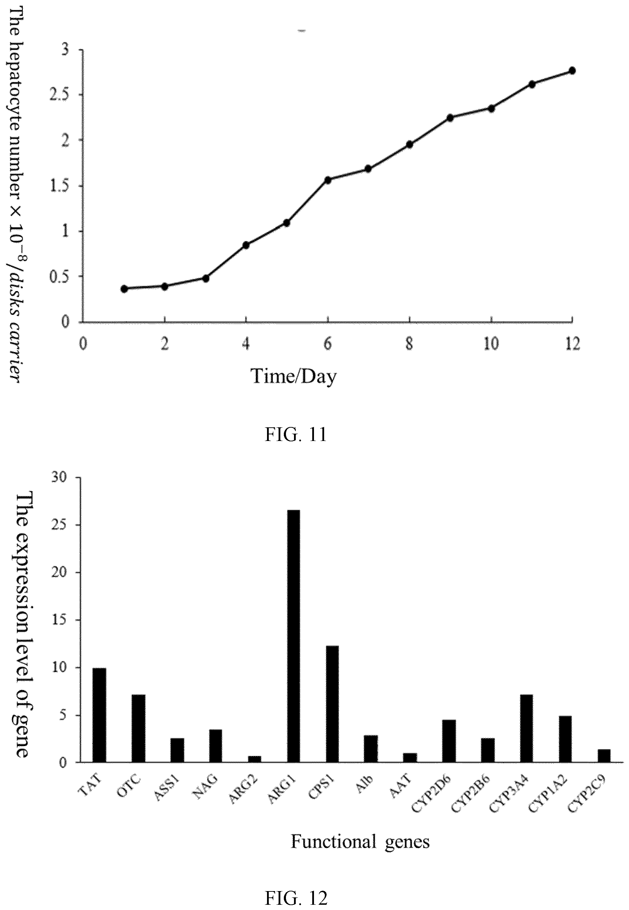

Referring to FIG. 11, the hepatic precursor-like cell line has a more regular morphological structure, which helps to exert favorable cell function.

The infected cell line constructed by the first construction method was used as the control cell line. The hepatic precursor-like cell line constructed by the first construction method was used as the first target cell line. The hepatic precursor-like cell line constructed by the second construction method was used as the second target cell line. The hepatic precursor-like cell line constructed by the third construction method was used as the third target cell line. The gene expression level, protein level, urea production capacity, ammonia clearance capacity, albumin synthesis capacity, and synthesis capacity of α1-antitrypsin (Alpha-1-antitrypsin, AAT) of different functional genes in the control cell line and each target cell line were analyzed and characterized.

Specifically, the RNAfast200 kit, produced by Shanghai Flytech Technology Co., Ltd. and catalog number 220010, was used to respectively extract the RNA of the control cell line and the first target cell line. Then, the RNA was reverse transcribed into cDNA by a reverse transcriptase, which was produced by Invitrogen Corporation, and the catalog number was 18064014. Finally, the relevantly functional genes of hepatocytes were expressed by fluorescence quantitative PCR to obtain a comparison diagram of the gene expression levels of the different functional genes of the control cell line and the first target cell line, shown in FIG. 2. The specific expression method of the fluorescent quantitative PCR was ordinary skills in the art and will not be repeated here.

The related functional genes were Albumin (Alb), AAT, Transferrin (TRF), Carbamoyl phosphate synthetase 1 (CPS1), Ornithine transcarbamylase (OTC), Arginino succinate synthetase 1 (ASS1), Arginase-1 (ARG1), Arginase-2 (ARG2), N-acetyl-beta-D Glucosaminidase (NAG) and 5 drug metabolism enzymes of phase I. The 5 drug metabolism enzymes of phase I were CTP3A4, CYP2D6, CYP2B6, CYP1A2, and CYP2C9.

Referring to FIG. 2, the gene levels of Alb, AAT, and TAT in the first target cell line were higher than the related gene levels in the control cell line. The result was provided that the first target cell line was overexpressed by FOAX3 with the excellent protein synthesis function. The gene levels of CPS1, OTC, ASS1, NAG, and ARG1 in the first target cell line were higher than the related gene levels in the control cell line, while the gene level of ARG2 was basically as same as the control cell line. The result was provided that the urea production capacity of the first target cell line was better than the control cell line. Therefore, the superior urea production capacity indicates that the first target cell line has the excellent detoxification function. The gene levels of CYP3A4, CYP1A2, and CYP2C9 in the first target cell line were higher than the related gene levels in the control cell line, while the gene level of CYP2D6 and CYP2B6 have the little difference with the gene level in the control cell line. The result was provided that the drug metabolism ability of the first target cell line was better than the control cell line.

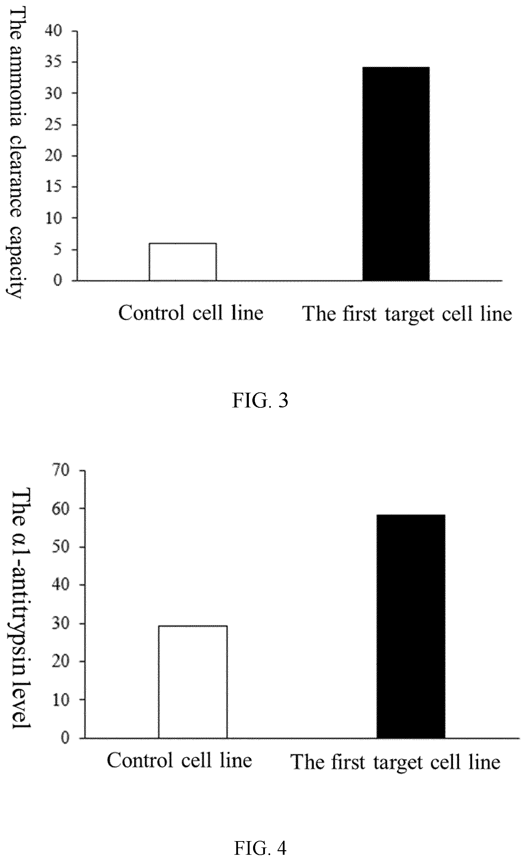

Further on, a rapid kit was produced by Megazyme Incorporation with the product number, K-AMIAR07/14. The rapid kit was used to characterize the ammonia clearance capacity of the first target cell line and the control cell line respectively. A comparison diagram of the ammonia clearance capacity was obtained and shown in FIG. 3.

Referring to FIG. 3, the physical meaning of the ammonia clearance level was the ammonia consumed by per 10⁶ cells per day. The ammonia clearance level of the control cell line was about 5.9, while the ammonia clearance level of the first target cell line was as high as 34.4, which was 6 times the ammonia clearance of the control cell line. As a result, the first target cell line possesses an excellent detoxification function.

Furthermore, an AAT ELISA kit was produced by Bethyl Incorporation with product number, E88-122. The AAT ELISA kit was used to detect the α1-antitrypsin level of the first target cell line and the control cell line respectively. A comparison diagram of the α1-antitrypsin level was obtained and shown in FIG. 4.

Referring to FIG.4, the physical meaning of α1-antitrypsin level was the nanogram mass of ATT synthesized by per 10⁶ cells per day. The α1-antitrypsin level of the control cell line was 29.2, while the α1-antitrypsin level of the first target cell line was as high as 58.5, which was 2 times the α1-antitrypsin level of the control cell line. The result was provided that the first target cell line possesses the good ATT synthesis capacity.

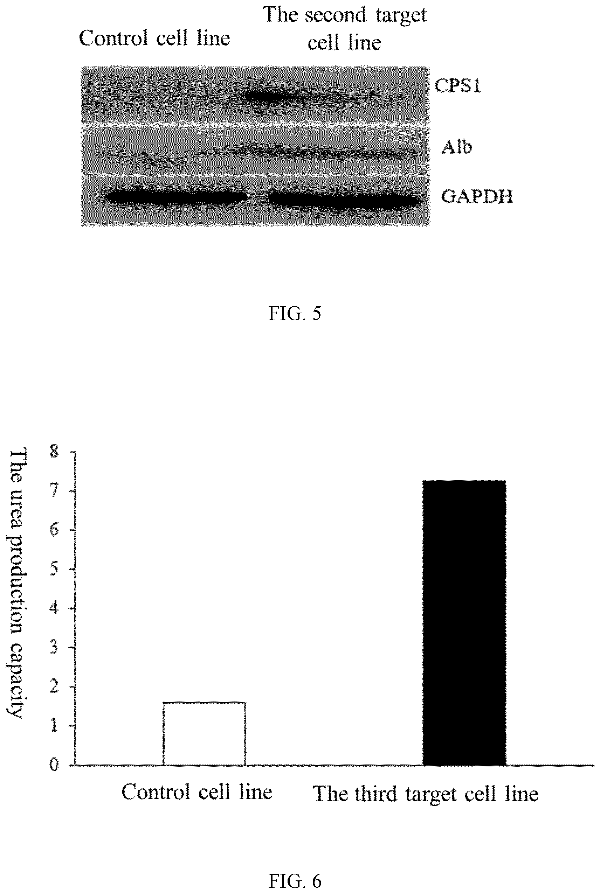

Taken Glyceraldehyde-3-phosphate dehydrogenase (GAPDH) as a reference, the protein level characterization of Alb and CPS1 of the second target cell line and the control cell line were measured by Western blot. Then, a comparison diagram of the protein levels was obtained and shown in FIG. 5.

To be more specifically, the specific implementation of the Western blotting method was mentioned in "Aggregation Culture Induces Functional Differentiation of Induced Hepatocyte-like Cells through Activation of Hippo Signaling" published in Cell Report, 2018, Vol. 25, 1.

Referring to FIG. 5, the GAPDH Western blotting of the second target cell line and the control cell line were not much different. By comparison with the GAPDH Western blotting, the Alb Western blot of the second target cell line was more significant than the Alb Western blot of the control cell line. The result was provided that the second target cell line has a better albumin synthesis function. Moreover, the CPS1 Western blotting of the second target cell line was more significant than the CPS1 Western blotting of the control cell line. The result was provided that the second target cell line has better urea production capacity than the control cell line.

A QuantiChrom^{™} Urea Assay Kit was produced by Bioassay System Incorporation with the catalog number, DIUR-500. The QuantiChrom^{™} Urea Assay Kit was used to characterize the urea production capacity of the third target cell line and the target cell line respectively. Then, a comparison diagram of the urea production levels was obtained and shown in FIG. 6. In FIG. 6, the physical meaning of the urea production level was the milligram of the urea produced by per 10⁶ cells per day.

Referring to FIG. 6, the urea production level of the control cell line was about 1.6, while the urea production level of the third target cell line was as much as 7.3, which was 5 times the urea production level of the control cell line. As a result, the third target cell line has excellent detoxification.

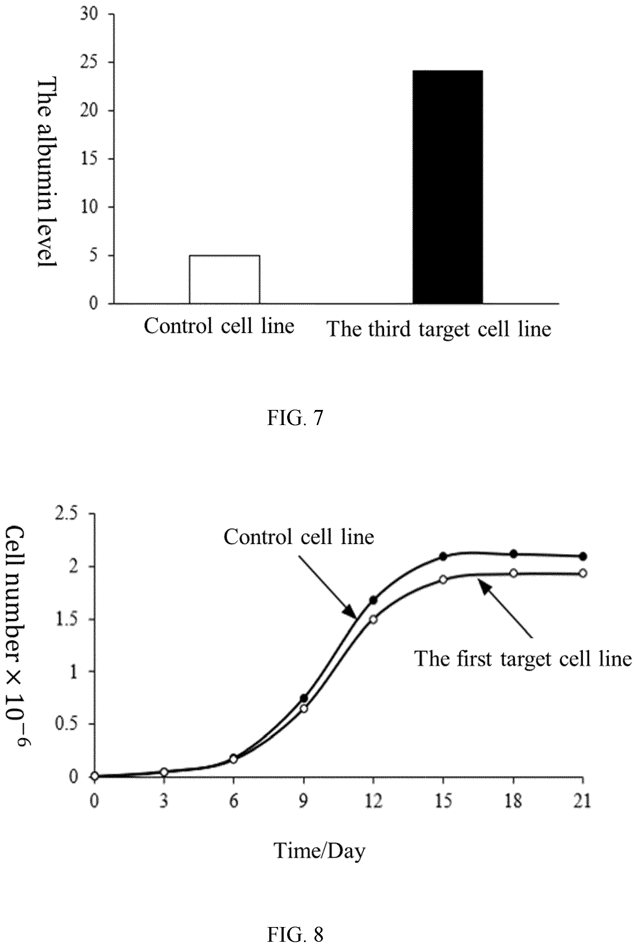

Further on, a human albumin ELISA kit was produced by Bethyl Incorporation with the product number, E88-129. The human albumin ELISA kit was used to detect the album levels of the third target cell line and the control cell line respectively. Then, a comparison diagram of the albumin level was obtained and shown in FIG. 7.

Referring to FIG. 7, the physical meaning of albumin level was the nanogram of Alb produced by per 10⁶ cells per day. The albumin level of the control cell line was about 5.0, while the albumin level of the third cell line was as high as 24.2, which was 5 times the albumin level of the control cell line. Therefore, the result was provided that the third target cell line has the superior albumin synthesis function.

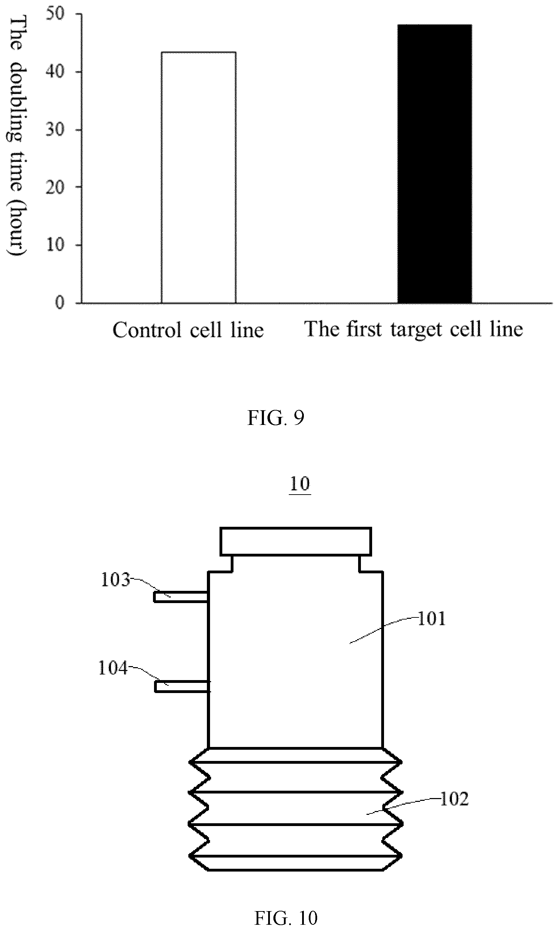

In the embodiments of this invention, the proliferation performance of the first target cell line and the control cell line were characterized. A comparison diagram of the proliferation performance shown in FIG. 8, and the comparison diagram of the doubling time shown in FIG. 9 were obtained.

Specifically, the specific characterized method of the proliferation performance was mentioned in the "Expansion and differentiation of human hepatocyte-derived liver progenitor-like cells for the study of hepatotropic pathogens" published in Cell Research, 2018, 29, 1.

Referring to FIG. 8, during the 21 days proliferation and growth period, the number of cells proliferated by the first target cell line and the control cell line and the growth rate were not with an obvious difference. Referring to FIG. 9, the doubling time of the first target cell line was about 48 hours, which was not much different from the doubling time of the control cell line of 43 hours. As a consequence, the growth characteristics of the first target cell line were affected by the overexpression of FOXA3 basically.

Also, the hepatic precursor-like cell line obtained by the construction methods was applied to the field of the bioartificial liver as an embodiment of this invention.

First, a bioartificial liver reactor was provided, referring to Embodiment 2 of the Chinese invention patent application number, CN106620916A, which seeding the hepatocytes on blank disks carriers. Specifically, the difference between the hepatocytes seeded process of CN106620916A and the Embodiment 2 disclosed was that the hepatocytes used in this application were constructed from the first construction method in Embodiment 1, that is, the first target cell line. The mass of the blank disk carriers was 11 g, and the hepatocyte number seeded per gram of the blank disk carriers was 1×10⁶ cells.

Subsequently, the hepatocytes seeded in the disk carriers were subjected to gas-liquid interactive cultured by the bioartificial liver reactor for 12 days, which induces the proliferation and growth of the hepatocytes.

FIG. 10 was a schematic diagram of the structure of the bioartificial liver reactor in some embodiments of this invention.

Referring to FIG. 10, the bioartificial liver reactor 10 includes a container body 101 and a corrugated pipe 102, as well as a liquid inlet pipe 103 and a liquid outlet pipe 104 penetrating the container body 101. The top of the container body 101 was provided with an air vent and an antibacterial breathable membrane to ensure corrugated pipe 102 elevated smoothly and block microorganisms in the air.

The bottom of the container body 101 had a gas-liquid channel, which was not marked in the FIG.10, to deliver 500 ml of the DMEM medium through the liquid inlet pipe 103 into the corrugated pipe 102. The disks carriers seeded with the hepatocytes were sealed in the container body 101, and the liquid inlet pipe 103 and the liquid outlet pipe 104 were closed. The corrugated pipe 102 was driven by an actuating device, which was not marked in the FIG.10 and set outside of the bioartificial liver reactor 10 to perform elevating movements periodically. Furthermore, the DMEM medium was driven to enter the container body 101 through the gas-liquid channel, so as to perform the gas-liquid interactive culturing to the disk carriers seeded with the hepatocytes.

In a specific period, the disks carriers seeded with the hepatocytes were immersed in the DMEM medium, and the liquid height was maintained unchanged. The liquid phase material exchange between hepatocytes and culture medium was performed for 60 seconds. After the liquid phase material exchange was completed, the liquid height of the DMEM medium was lower, so that the hepatocytes and the oxygen in the air perform a gas phase material exchange.

After the gas-liquid interactive culture was completed, the liquid outlet pipe 104 was opened, and the medium was discharged through the compressed liquid outlet pipe 104 and the corrugated pipe 102.

In the process of the gas-liquid interactive culture, 3 pieces of the disk carriers seeded with the hepatocytes were taken out of the container body 101 every day to detect the number of hepatocytes. Therefore, a schematic diagram of the proliferation curve shown in FIG. 11 was obtained. The ordinate shown in FIG. 11 represents the number of hepatocytes seeded in the disks carriers per unit mass.

Referring to FIG. 11, during the process of the gas-liquid interactive culture for 12 days, the linear relationship between the number of hepatocytes and the culture time was obvious. Moreover, the number of hepatocytes was significant and the growth trend was stable.

The aforementioned corresponding characterization methods to analyze and characterize the gene expression functional genes in the hepatocytes seeded in the disks carriers after completing the gas-liquid interactive culture to obtain FIG. 12, which was a schematic diagram of the gene expression level of the different functional genes.

Referring to FIG. 12, the hepatic precursor-like cell line obtained by the first construction method were acquired the significant expression in the related functional genes, such as Alb, AAT, CPS1, OTC, ASS1, ARG1, ARG2, NAG, CYP3A4, CYP2D6, CYP2B6, CYP1A2, CYP2C9, and Trans-activating Protein (TAT).

The aforementioned corresponding characterization methods to characterize the albumin synthesis capacity, AAT synthesis capacity, urea production capacity, and ammonia clearance capacity of the hepatocytes seeded in the disks carriers after completing the gas-liquid interactive culture were characterized. The results indicates that the mass of 71.7 ng of Alb produced per day, 163.9 ng of α1-antitrypsin produced per day, 69.7 µg of ammonia consumed per day, and 25.2 mg of urea produced per day for every 10⁶ hepatocytes seeded in the disks carriers complete the gas-liquid interactive culture.

As the mentioned analysis above, the hepatic precursor-like cell line obtained by the first construction method acquires good albumin synthesis function, AAT synthesis function, detoxification function, and drug metabolism ability, after the gas-liquid interactive culture in the bioartificial liver reactor 10.

## Claims

1. A method for producing a hepatic precursor-like cell line, comprising:
S1: providing human primary hepatocyte cultures, proliferation-and passage-culturing the human primary hepatocyte cultures with a passage ratio of 1:3 to 1:6 and a passage number of 2 to 30 to obtain to-be-infected cultures;
S2: confluence-culturing the to-be-infected cultures at a seeding density of 0.5×10⁴-1×10⁵ cells/cm² to obtain adherent cells, wherein the confluence rate of the adherent cells is not less than 80%;
S3: virus-infecting the adherent cells with a lentivirus suspension and a viral infection enhancement buffer using TEM medium, wherein one expressed gene of the lentivirus is SV40 large T antigen, HPV E6E7 gene or human telomerase reverse transcriptase gene (hTERT gene), and the viral infection enhancement buffer is polybrene buffer, and replacing the TEM medium during the virus infection, wherein the amount of the lentivirus in the suspension is 0.5 to 50 times the amount of adherent cells, and the concentration of the virus infection enhancement buffer is 6-12 µg/ml;
S4: proliferation-culturing and subculturing the infected cell with a selecting agent and an amplification medium for a proliferation duration of 24-36 hours to obtain immortalized cells with a subculture ratio of 1:3 to 1:6 and a passage number of 10 to 100, wherein the selecting agent is puromycin, hygromycin, or neomycin sulfate and used for selection of the infected cell, and the amplification medium is the TEM medium; and
S5: overexpressing FOXA3 in the immortalized cell line to obtain the hepatic precursor-like cell line.

2. The method according to claim 1, wherein, in the step S1, the human primary hepatocyte cultures are separated from the normal liver tissues of the donor by a collagenase perfusion method.

3. The method according to claim 2, wherein, in the step S1, the human primary hepatocyte cultures are proliferation-cultured at a seeding density of 0.5×10⁴-2×10⁴ cells/cm² for 6 to 12 days.

4. The method according to claim 1, wherein, in the step S2, performing the confluence-culturing to the to-be-infected cells in a culture dish coated with a coating buffer at a coating density of 0.87-1.74 µl/cm² in advance for at least 24 hours.

5. The method according to claim 4, wherein, in the step S3, the lentivirus suspension and the viral infection enhancement buffer are added into the culture dish with the adherent cells, and after 6 to 12 hours, the TEM medium is replaced.

6. The method according to claim 5, wherein, after replacing the TEM medium, the adherent cells are continuously cultured for 24 to 72 hours to complete the virus infection.

7. A hepatic precursor-like cell line prepared by the method of any one of claims 1-6, wherein, the hepatic precursor-like cell line is preserved in China Center for Type Culture Collection with a preservation number of CCTCC NO. C2019120 and has a category name of ALI-CELL-85F.

8. Use of the hepatic precursor-like cell line prepared by the method of any one of claims 1-7 in a bioartificial liver reactor comprising a container body (101), a corrugated pipe (102), a liquid inlet pipe (103) and a liquid outlet pipe (104) penetrating the container body (101), and wherein the top of the container body (101) is provided with an air vent and an antibacterial breathable membrane configured to ensure that the corrugated pipe (102) is elevated and blocks microorganisms.

## Patentansprüche

1. Verfahren zur Herstellung einer hepatischen vorläuferähnlichen Zellinie, umfassend:
S1: Bereitstellung von menschlichen primären Hepatozytenkulturen, Proliferation und Durchgangskultivierung der menschlichen primären Hepatozytenkulturen mit einem Durchgangsverhältnis von 1:3 bis 1:6 und einer Durchgangszahl von 2 bis 30, zur Gewinnung von zu infizierenden Kulturen;
S2: Konfluenz-Kultivierung der zu infizierenden Kulturen mit Saatdichte von 0.5 × 10⁴-1 × 10⁵ Zellen/cm², um adhärente Zellen zu erhalten, wobei die Konfluenzrate der adhärenten Zellen nicht weniger als 80 % beträgt;
S3: Infektion mit einem Virus der anhaftenden Zellen durch einer Lentivirus-Suspension und einem viralen Infektionsverstärkungspuffer unter Verwendung von TEM-Medium, wobei ein exprimiertes Gen des Lentivirus das große SV40-T-Antigen, das HPV-E6E7-Gen oder das menschliche Telomerase-Reverse-Transkriptase-Gen (hTERT-Gen) ist, und der virale Infektionsverstärkungspuffer Polybrenpuffer ist, und das TEM-Medium während der Virusinfektion ersetzt, wobei die Menge des Lentivirus in der Suspension das 0.5- bis 50-fache der Menge an adhärenten Zellen beträgt und die Konzentration des Virusinfektionsverstärkungspuffers 6-12 µg/ml beträgt;
S4: Proliferation-Kultivierung und Subkultivierung der infizierten Zelle mit einem Selektionsmittel und einem Amplifikationsmedium für eine Proliferationsdauer von 24-36 Stunden, zur Gewinnung von immortalisierten Zellen mit einem Subkulturverhältnis von 1:3 bis 1:6 und einer Durchgangszahl von 10 bis 100, wobei das Selektionsmittel Puromycin, Hygromycin oder Neomycinsulfat ist und es zur Selektion der infizierten Zelle verwendet wird und das Amplifikationsmedium das TEM-Medium ist; und
S5: Überexpression von FOXA3 in der immortalisierten Zelllinie, zur Gewinnung einer hepatischen vorläuferähnlichen Zelllinie.

2. Verfahren nach Anspruch 1, wobei in Schritt S1, die menschlichen primären Hepatozytenkulturen von den normalen Lebergeweben des Spenders durch ein Kollagenase-Perfusionsverfahren getrennt werden.

3. Verfahren nach Anspruch 2, wobei in Schritt S1 die menschlichen primären Hepatozytenkulturen mit einer Saatdichte von 0.5 × 10⁴-2 × 10⁴ Zellen/cm² für 6 bis 12 Tage in einer Proliferationskultur kultiviert werden.

4. Verfahren nach Anspruch 1, wobei in Schritt S2 die Konfluenz-Kultivierung der zu infizierenden Zellen in einer Kultivierungsschale durchgeführt wird, die mit einem Beschichtungspuffer mit einer Beschichtungsdichte von 0.87 bis 1.74 µ/cm² im Voraus für mindestens 24 Stunden beschichtet wurde.

5. Verfahren nach Anspruch 4, wobei in Schritt S3 die Lentivirus-Suspension und der virale Infektionsverstärkungspuffer in die Kultivierungsschale mit den anhaftenden Zellen hinzugefügt werden und nach 6 bis 12 Stunden das TEM-Medium ersetzt wird.

6. Verfahren nach Anspruch 5, wobei nach dem Austausch des TEM-Mediums, die anhaftenden Zellen durchgehend für 24 bis 72 Stunden kultiviert werden, bis die Virusinfektion vollständig ist.

7. Hepatische vorläuferähnliche Zellinie, hergestellt nach einem der Ansprüche 1 bis 6, wobei die hepatische vorläuferähnliche Zellinie im Chinesischen Zentrum für Typkulturensammlung aufbewahrt wird, mit der Konservierungsnummer CCTCC NO. C2019120 und der Kategoriebezeichnung ALI-CELL-85F.

8. Verwendung der hepatischen vorläuferähnlichen Zellinie, hergestellt nach einem der Ansprüche 1 bis 7, in einem Reaktor für eine bioartifizielle Leber, umfassend einen Behälterkörper (101), ein Wellrohr (102), ein Flüssigkeitseinlassrohr (103) und ein Flüssigkeitsauslassrohr (104), die den Behälterkörper (101) durchdringen, und wobei die Oberseite des Behälterkörpers (101) mit einer Entlüftungsöffnung und einer antibakteriellen, atmungsaktiven Membran versehen ist, mit der sichergestellt wird, dass das Wellrohr (102) erhöht ist und Mikroorganismen blockiert.

## Revendications

1. Procédé de production d'une lignée cellulaire de type précurseur hépatique, comprenant :
S1 : fournir des cultures d'hépatocyte primaire humain, effectuer une prolifération et une culture de passage sur les cultures d'hépatocyte primaire humain à un rapport de passage de 1 : 3 à 1 : 6 et un nombre de passage de 2 à 30 pour obtenir des cultures à infecter ;
S2 : effectuer une culture de confluence sur les cultures à infecter à une densité d'ensemencement de 0,5×10⁴-1×10⁵ cellules/cm² pour obtenir des cellules adhérentes, dans lequel le taux de confluence des cellules adhérentes n'est pas inférieur à 80 % ;
S3 : effectuer une infection virale sur les cellules adhérentes avec une suspension de lentivirus et un tampon de renforcement de l'infection virale en utilisant un milieu TEM, dans lequel un gène exprimé du lentivirus est le antigène T large SV40, le gène HPV E6E7 ou le gène de transcriptase inverse de la télomérase humaine (gène hTERT), et le tampon de renforcement de l'infection virale est un tampon en polybrène, et renouveler le milieu TEM pendant l'infection virale, dans lequel la quantité de lentivirus dans la suspension est de 0,5 à 50 fois la quantité de cellules adhérentes, et la concentration de tampon de renforcement de l'infection virale est de 6 à 12 µg/ml ;
S4 : effectuer une culture de prolifération et une sous-culture sur les cellules infectées avec un agent de sélection et un milieu d'amplification pendant une durée de prolifération de 24 à 36 heures afin d'obtenir des cellules immortalisées à un rapport de sous-culture de 1 : 3 à 1:6 et un nombre de passage de 10 à 100, dans lequel l'agent de sélection est choisi parmi la puromycine, l'hygromycine ou le sulfate de néomycine et utilisé pour la sélection des cellules infectées, et le milieu d'amplification est le milieu TEM ; et
S5 : sur-exprimer FOXA3 dans la lignée cellulaire immortalisée pour obtenir la lignée cellulaire de type précurseur hépatique.

2. Procédé selon la revendication 1, dans lequel, à l'étape S1, les cultures d'hépatocyte primaire humain sont séparées des tissus hépatiques normaux du donneur par un procédé de perfusion de collagénase.

3. Procédé selon la revendication 2, dans lequel, à l'étape S1, les cultures d'hépatocyte primaire humain sont soumises à une culture de prolifération à une densité d'ensemencement de 0,5×10⁴-2× 10⁴ cellules/cm²pendant 6 à 12 jours.

4. Procédé selon la revendication 1, dans lequel, à l'étape S2, effectuer à l'avance la culture de confluence sur les cultures à infecter dans une boîte de culture recouverte d'un tampon d'enrobage à une densité d'enrobage de 0,87-1,74 µl/cm² pendant au moins 24 heures.

5. Procédé selon la revendication 4, dans lequel, à l'étape S3, la suspension de lentivirus et le tampon de renforcement de l'infection virale sont ajoutés dans la boîte de culture avec les cellules adhérentes, et le milieu TEM est renouvelé à la fin de 6 à 12 heure.

6. Procédé selon la revendication 5, dans lequel, après avoir renouvelé le milieu TEM, les cellules adhérentes sont cultivées en continu pendant 24 à 72 heures pour compléter l'infection virale.

7. Lignée cellulaire de type précurseur hépatique préparée par le procédé selon l'une quelconque des revendications 1 à 6, dans laquelle la lignée cellulaire de type précurseur hépatique est conservée au Centre chinois pour la collection de la culture typographique sous le numéro de conservation CCTCC NO. C2019120 et a un nom de catégorie ALI-CELL-85F.

8. Utilisation de la lignée cellulaire de type précurseur hépatique préparée par le procédé selon l'une quelconque des revendications 1 à 7 dans un réacteur hépatique bioartificiel qui comprend un corps de récipient (101), un tuyau ondulé (102), un tuyau d'entrée de liquide (103) et un tuyau de sortie de liquide (104) pénétrant dans le corps de récipient (101), dans laquelle la partie supérieure du corps de récipient (101) est pourvu d'un évent et d'une membrane respiratoire antibactérienne configurée pour garantir une élévation du tuyau ondulé (102) et un blocage des micro-organismes.
